# EUROPÄISCHE PATENTSCHRIFT

(11) **EP 3 079 591 B1**
(45) Veröffentlichungstag und Bekanntmachung des Hinweises auf die Patenterteilung: **31.03.2021**
(21) Anmeldenummer: 15706728.1
(22) Anmeldetag: 16.02.2015
(51) Int. Cl.: A61B 6/00

(54) **COMPUTERTOMOGRAFIEANLAGE UND PATIENTENTISCH MIT EINER BERÜHRUNGSLOSEN ÜBERTRAGUNG ELEKTRISCHER SIGNALE**
COMPUTER TOMOGRAPHY INSTALLATION AND PATIENT TABLE HAVING CONTACTLESS TRANSMISSION OF ELECTRICAL SIGNALS
SYSTÈME DE TOMODENSITOMÉTRIE ET TABLE D'EXAMEN DOTÉE D'UNE TRANSMISSION SANS CONTACT DE SIGNAUX ÉLECTRIQUES

(30) Priorität: 02.04.2014 DE 102014206295
(43) Veröffentlichungstag der Anmeldung: 19.10.2016
(73) Patentinhaber: Siemens Healthcare GmbH, 91052 Erlangen (DE)
(72) Erfinder: MODEL, Volker, 90766 Fürth (DE)
(86) Internationale Anmeldenummer: PCT/EP2015/053186
(87) Internationale Veröffentlichungsnummer: WO 2015/149983

(56) Entgegenhaltungen:
- DE-A1- 19 533 819
- DE-A1- 19 533 821
- DE-A1-102006 036 420

## Beschreibung

### Gebiet der Erfindung

Die Erfindung betrifft eine Computertomografieanlage und einen Patiententisch mit einer berührungslosen Übertragung von elektrischen Signalen zwischen zueinander bewegbaren Einheiten.

### Hintergrund der Erfindung

Für viele Anwendungen in der Medizintechnik müssen elektrische Signale bzw. Daten von einem bewegten medizintechnischen Anlagenteil zu einem ruhenden medizintechnischen Anlagenteil und umgekehrt übertragen werden.

Ein bevorzugtes Anwendungsgebiet der vorliegenden Erfindung betrifft die Datenübertragung zwischen dem rotierenden und dem stationären Teil einer Computertomografieanlage. Beim Betrieb der Computertomografieanlage müssen die von den Röntgendetektoren erfassten Daten vom rotierenden Teil an den stationären Teil der Computertomografieanlage übertragen werden, um sie dort weiterzuverarbeiten. Dabei sind große Datenmengen in kurzer Zeit zu übertragen.

Bei vielen derzeit verfügbaren Computertomografieanlagen wird ein berührungsloses "Schleifring"-System zur Datenübertragung eingesetzt, wie es beispielsweise aus der Patentschrift US 5140696 A bekannt ist. Das dort beschriebene Datenübertragungssystem umfasst eine Sendeeinheit am rotierenden Teil sowie eine Empfangseinheit am stationären Teil. Die Sendeeinheit weist zumindest eine mit einem Sender verbundene Hochfrequenzleitung als Sendeantenne auf, die am Umfang des rotierenden Teils eines Drehrahmens angeordnet ist. Die Empfangseinheit umfasst einen Empfänger und zumindest eine mit dem Empfänger verbundene Empfangsantenne, die durch einen kurzen Abschnitt einer Hochfrequenzleitung gebildet ist. Beim Betrieb der Computertomografieanlage bewegt sich die Sendeantenne in geringem Abstand an der am stationären Teil befestigten Empfangsantenne vorbei, so dass die auf der sendenden Hochfrequenzleitung propagierenden Signale über das Nahfeld in die Empfangsantenne einkoppeln.

Die Offenlegungsschrift DE 102 06 160 A1 offenbart eine derartige Signalübertragung zwischen bewegten Teilen, wobei die Hochfrequenzleitung als Streifenleitung mit einem Dielektrikum ausgeführt ist.

Die Offenlegungsschrift DE 195 33 819 A1 offenbart ein Computertomografiesystem mit einem geschlitzten koaxialen elektrischen Leiter zur berührungslosen Signalübertragung, wobei das elektrische Signal durch den Innenleiter eines Koaxialkabels, der in den Schlitz des Koaxialleiters eingreift, ausgekoppelt wird.

### Zusammenfassung der Erfindung

Es ist Aufgabe der Erfindung, eine Computertomografieanlage und einen Patiententisch anzugeben, die eine elektrische Datenübertragung mit hoher Datenübertragungskapazität und geringer Fernfeldstörung ermöglichen.

Gemäß der Erfindung wird die gestellte Aufgabe mit der Computertomografieanlage und dem Patiententisch der unabhängigen Patentansprüche gelöst. Vorteilhafte Weiterbildungen sind in den abhängigen Ansprüchen angegeben.

Erfindungsgemäß wird zwischen zueinander relativ bewegten Teilen einer Computertomografieanlage oder eines Patiententisches eine berührungslose Signalübertragung mittels längsgeschlitzter koaxialer Leiterelemente, sogenannter Koaxialleiter, durchgeführt. Das zu übertragende Datensignal wird auf einen Hochfrequenzträger aufmoduliert. Die kontaktlose Übertragung findet dann schmalbandig (Modulationsbandbreite bis etwa 30% der Hochfrequenzträgerfrequenz) mittels der längsgeschlitzten koaxial aufgebauten Leitungskopplerstruktur statt. Die Übertragungsfunktion der Kopplerstruktur selbst ist breitbandig mit einem entsprechendem Frequenzgang (Hochpassverhalten). Eine Koaxialleitung (z.B. der Sendekoppler) deckt den gesamten Verfahrbereich, bei rotierenden Teilen den Kreisumfang, ab. Eine zweite kurze Koaxialleitung (z.B. der Empfangskoppler) koppelt über ihre Strecke das modulierte Hochfrequenzträgersignal aus. Die Länge der Koppelstrecke wird von der Trägerfrequenz und der Modulationsbandbreite bestimmt.

Die Erfindung beansprucht eine Computertomografieanlage mit einer berührungslosen Datensignalübertragung, aufweisend:
- mindestens einen längsgeschlitzten Koaxialleiter,
- mindestens eine Hochfrequenzsendeeinheit, die ein mit einem zu übertragenden Datensignal moduliertes Hochfrequenzträgersignal in den Koaxialleiter einspeist,
- mindestens einen längsgeschlitzten koaxialen Koppelleiter, der ausgebildet ist, aus dem Nahfeld des Leiterelements das abgestrahlte modulierte Hochfrequenzträgersignal zu empfangen, und
- mindestens eine Hochfrequenzempfangseinheit, die mit dem Koppelleiter elektrisch verbunden ist und ausgebildet ist, das Datensignal aus dem empfangenen modulierten Hochfrequenzträgersignal zu extrahieren,
- wobei der Koaxialleiter auf einem rotierenden oder einem feststehenden Gantryteil und der Koppelleiter korrespondierend auf dem feststehenden oder dem rotierenden Gantryteil angeordnet ist.

Die Erfindung bietet aufgrund der konstruktiven Eigenschaften der längsgeschlitzten Koaxialleitungen ein hervorragendes Verhalten hinsichtlich der Fernfelddämpfung, was zu einer hohen Störfestigkeit und zu einer sehr geringen Emission des modulierten Signals führt. Die Datenübertragung eignet sich besonders für eine Übertragung im ein- und zweistelligen Gigahertz-Bereich. Der Leitungskoppler kann kostengünstig aus einer Koaxialleitung durch teilweises Abisolieren oder durch einen in eine Nut eingebetteten isolierten Innenleiter mit definierter Wellenimpedanz realisiert werden.

In einer Weiterbildung der Erfindung kann die Frequenz des Hochfrequenzträgersignals größer 10 GHz sein. Bevorzugt kann im K- und Ka-Band bei 18 bis 35 GHz übertragen werden. Höhere Frequenzen bis 60 GHz sind möglich, führen aber zu einer Reduzierung des koaxialen Querschnitts.

In einer weiteren Ausführungsform umfasst die Vorrichtung ein erstes Trägerelement, in dem der Koaxialleiter ausgebildet ist.

In einer weiteren Ausführungsform kann das erste Trägerelement aus Metall sein und einen ersten Außenleiter des Koaxialleiters bilden. Der Querschnitt des ersten Trägerelements ist bevorzugt rechteckig und in einer Nut des ersten Trägerelements ist ein erster Innenleiter angeordnet.

In einer Weiterbildung umfasst die Vorrichtung ein zweites Trägerelement, in dem der koaxiale Koppelleiter ausgebildet ist.

In einer weiteren Ausbildung ist das zweite Trägerelement aus Metall und bildet einen zweiten Außenleiter des koaxialen Koppelleiters. Der Querschnitt des zweiten Trägerelements ist bevorzugt rechteckig und in einer Nut des ersten Trägerelements ist ein zweiter Innenleiter angeordnet.

Außerdem beanspruche die Erfindung einen Patiententisch mit einer koaxialen Anordnung wie vorgehend beschrieben für die Datensignalübertragung zwischen translatorisch bewegten Teilen.

Weitere Besonderheiten und Vorteile der Erfindung werden aus den nachfolgenden Erläuterungen eines Ausführungsbeispiels anhand von schematischen Zeichnungen ersichtlich.

Es zeigen:
- Fig. 1:: einen Querschnitt durch ein koaxiales Leiterelement,
- Fig. 2:: einen Querschnitt durch eine Vorrichtung mit einem koaxialen Leiterelement und einem koaxialen Koppelleiterelement,
- Fig. 3:: ein Blockschaltbild einer Vorrichtung mit einem rotierbaren koaxialen Leiterelement,
- Fig. 4:: ein Blockschaltbild einer Vorrichtung mit einem rotierbaren segmentierten koaxialen Leiterelement,
- Fig. 5:: ein Blockschaltbild einer Vorrichtung mit einem translatorisch verschiebbaren koaxialen Leiterelement,
- Fig. 6:: ein Blockschaltbild einer Vorrichtung mit einem feststehenden koaxialen Leiterelement und
- Fig. 7:: eine Computertomografieanlage.

### Detaillierte Beschreibung eines Ausführungsbeispiels

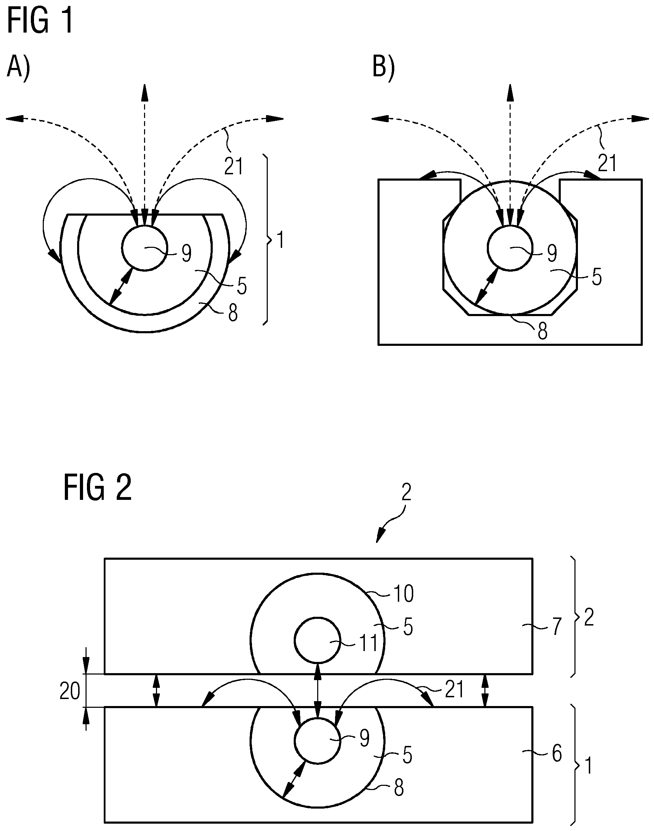

**Fig. 1** zeigt Querschnitte durch ein längsgeschlitztes koaxiales Leiterelement 1 (= Koaxialleiter). Der Querschnitt A) zeigt ein Koaxialkabel und der Querschnitt B) zeigt ein Koaxialkabel, dem der Außenleiter entfernt wurde und das in eine Fräsnut eingelegt ist. Die Längsschlitzung kann durchgängig oder periodisch beabstandet ausgeführt sein. Das Leiterelement 1 umfasst einen ersten Innenleiter 9 und einen ersten Außenleiter 8. Zwischen dem ersten Außenleiter 8 und dem ersten Innenleiter 9 befindet sich ein Dielektrikum 5, das den ersten Innenleiter 9 koaxial zum ersten Außenleiter 8 fixiert. Der erste Außenleiter 8 ist nach oben offen, d.h. aufgeschlitzt. Durch den sich bildenden Spalt kann eine im Leiterelement 1 geführte elektrische Welle teilweise entweichen. Das entstehende elektrische Feld ist durch die Feldlinien 21 dargestellt. In Querschnitt B) füllt das Dielektrikum die Fräsnut nicht vollständig aus.

**Fig. 2** zeigt was passiert, wenn in das Nahfeld des geschlitzten koaxialen Leiterelements 1 ein längsgeschlitztes koaxiales Koppelleiterelement 2 (= koaxialer Koppelleiter) gebracht wird. Aus dem Nahfeld koppelt elektrische Energie in das Koppelleiterelement 2, beispielweise auch ein Koaxialkabel, über. Das elektromagnetische Feld wird durch seine Feldlinien 21 dargestellt.

**Fig. 2** zeigt einen Querschnitt durch das Leiterelement 1 und durch das dazu mit dem Luftspalt 20 beabstandet angeordnete Koppelleiterelement 2. Das Leiterelement 1 umfasst ein elektrisch leitfähiges erstes Trägerelement 6, das den ersten Außenleiter 8 bildet. In das erste Trägerelement 6 ist der erste Innenleiter 9 mit dem Dielektrikum 5 eingepresst. Das Koppelleiterelement 2 umfasst ein elektrisch leitfähiges zweites Trägerelement 7, das den zweiten Außenleiter 10 bildet. In das zweite Trägerelement 7 ist der zweite Innenleiter 11 mit dem Dielektrikum 5 eingepresst. Der Luftspalt 20 ist nur wenige Millimeter groß.

Die knapp übereinander liegenden elektrisch leitfähigen Trägerelemente 6 und 7 wirken kapazitiv und bilden für hohe Frequenzen einen Kurzschluss. Eine Signalübertragung ist nur zwischen dem ersten und dem zweiten Innenleiter 9, 11 möglich.

Das elektrische Leiterelement 1 und das elektrische Koppelleiterelement 2 haben beispielsweise einen Durchmesser der Außenleiter 8, 10 von etwa 5,5 mm und der Innenleiter 9, 11 von etwa 1,5 mm. Die Trägerelemente 6, 7 sind beispielsweise aus Kupfer und die Innenleiter 9, 11 aus silberbeschichtetem Kupfer. Das Dielektrikum 5 ist zum Beispiel aus PTFE. Übertragungen mit einer Trägerfrequenz bis 35 GHz sind damit möglich. Für höhere Frequenzen muss der koaxiale Querschnitt reduziert werden, um den Koaxialleiter frei von Moden zu halten. Dadurch reduziert sich auch der mögliche Abstand für die Kopplerstruktur.

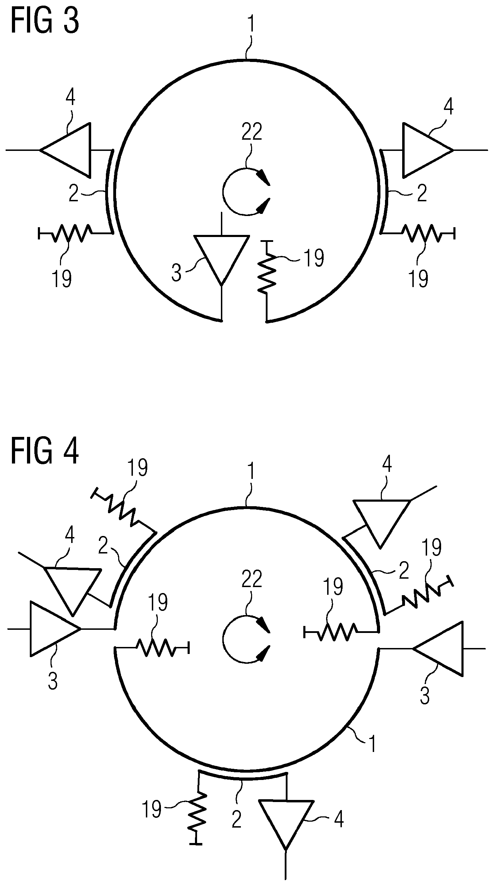

**Fig. 3** zeigt ein Blockschaltbild einer Vorrichtung mit einem rotierbaren koaxialen Leiterelement 1. In das Leiterelement 1 wird an seinem ersten Ende mittels der Hochfrequenzsendeeinheit 3 ein mit dem Datensignal moduliertes Hochfrequenzträgersignal eingespeist. Das Leiterelement 1 ist entsprechend **Fig. 2** aufgebaut und an seinem zweiten Ende mit einer Terminierung 19, beispielweise eine verlustbehaftete Leitung oder ein Abschlusswiderstand, zum reflexionsfreien Abschluss versehen. Das Leiterelement 1 ist in der Drehrichtung 22 rotierbar gelagert.

Zum Auskoppeln des Hochfrequenzträgersignals aus dem ersten Leiterelement 1 sind einander gegenüberliegend zwei Koppelleiterelemente 2, aufgebaut entsprechend **Fig. 2****,** entlang des Umfangs des Leiterelements 1 angeordnet. Das Koppelleiterelement 2 ist vorzugsweise wenige Wellenlängen (ein paar cm) lang und mit einer Terminierung 19 an seinem ersten Ende abgeschlossen. An seinem zweiten Ende ist eine Hochfrequenzempfangseinheit 4 angeschlossen, die das ausgekoppelte Hochfrequenzträgersignal empfängt und demoduliert.

Optional kann auch das Koppelleiterelement 2 rotierbar und das Leiterelement 1 fest angeordnet sein.

**Fig. 4** zeigt ein Blockschaltbild einer Vorrichtung mit einem in zwei gleich lange Segmente geteilten rotierbaren koaxialen Leiterelement 1. In die beiden Leiterelemente 1 wird an ihren ersten Enden mittels der Hochfrequenzsendeeinheit 3 ein mit dem Datensignal moduliertes Hochfrequenzträgersignal eingespeist. Die Leiterelemente 1 sind entsprechend **Fig. 2** aufgebaut und an ihren zweiten Enden mit einer Terminierung 19 zum reflexionsfreien Abschluss versehen. Die Leiterelemente 1 sind in der Drehrichtung 22 rotierbar gelagert.

Zum Auskoppeln des Hochfrequenzträgersignals aus dem ersten Leiterelement 1 ist ein Koppelleiterelement 2, aufgebaut entsprechend **Fig. 2****,** entlang des Umfangs der Leiterelemente 1 angeordnet. Das Koppelleiterelement 2 ist vorzugsweise wenige Wellenlängen (ein paar cm) lang und mit einer Terminierung 19 an seinem ersten Ende abgeschlossen. An seinem zweiten Ende ist eine Hochfrequenzempfangseinheit 4 angeschlossen, die das ausgekoppelte Hochfrequenzträgersignal empfängt und demoduliert.

Optional kann auch das Koppelleiterelement 2 rotierbar und das Leiterelement 1 fest angeordnet sein.

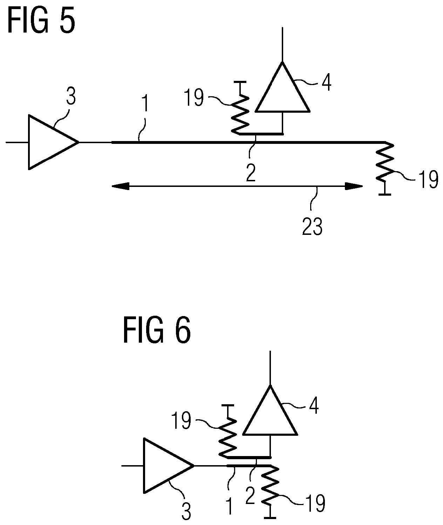

**Fig. 5** zeigt ein Blockschaltbild einer Vorrichtung mit einem translatorisch verschiebbaren koaxialen Leiterelement 1. In das Leiterelement 1 wird an seinem ersten Ende mittels der Hochfrequenzsendeeinheit 3 ein mit dem Datensignal moduliertes Hochfrequenzträgersignal eingespeist. Das Leiterelement 1 ist entsprechend **Fig. 2** aufgebaut und an seinem zweiten Ende mit einer Terminierung 19 zum reflexionsfreien Abschluss versehen. Das Leiterelement 1 ist in der Bewegungsrichtung 23 verschiebbar gelagert.

Zum Auskoppeln des Hochfrequenzträgersignals aus dem ersten Leiterelement 1 ist ein Koppelleiterelement 2, aufgebaut entsprechend **Fig. 2****,** entlang des Leiterelements 1 angeordnet. Das Koppelleiterelement 2 ist vorzugsweise wenige Wellenlängen (ein paar cm) lang und mit einer Terminierung 19 an seinem ersten Ende abgeschlossen. An seinem zweiten Ende ist eine Hochfrequenzempfangseinheit 4 angeschlossen, die das ausgekoppelte Hochfrequenzträgersignal empfängt und demoduliert.

Optional kann auch das Koppelleiterelement 2 bewegbar und das Leiterelement 1 fest angeordnet sein.

**Fig. 6** zeigt ein Blockschaltbild einer Vorrichtung mit einem feststehenden koaxialen Leiterelement 1. In das Leiterelement 1 wird an seinem ersten Ende mittels der Hochfrequenzsendeeinheit 3 ein mit dem Datensignal moduliertes Hochfrequenzträgersignal eingespeist. Das Leiterelement 1 ist entsprechend **Fig. 2** aufgebaut und an seinem zweiten Ende mit einer Terminierung 19 zum reflexionsfreien Abschluss versehen.

Zum Auskoppeln des Hochfrequenzträgersignals aus dem ersten Leiterelement 1 ist ein Koppelleiterelement 2, aufgebaut entsprechend **Fig. 2****,** entlang des Leiterelements 1 angeordnet. Das Koppelleiterelement 2 ist vorzugsweise wenige Wellenlängen (ein paar cm) lang und mit einer Terminierung 19 an seinem ersten Ende abgeschlossen. An seinem zweiten Ende ist eine Hochfrequenzempfangseinheit 4 angeschlossen, die das ausgekoppelte Hochfrequenzträgersignal empfängt und demoduliert.

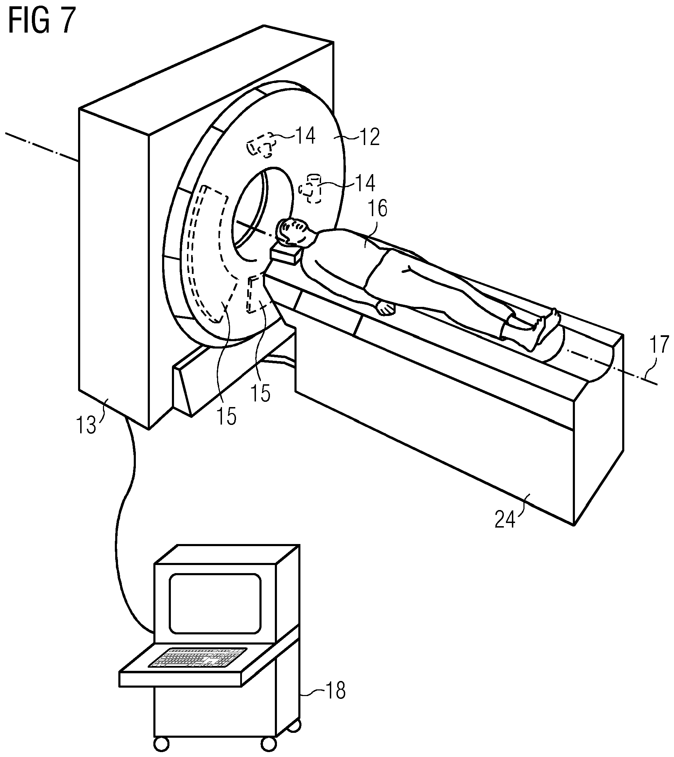

**Fig. 7** zeigt eine erfindungsgemäße Computertomographieanlage mit einem feststehendem Gantryteil 13, in dem sich ein rotierbarer Gantryteil 12 befindet, auf dem zwei Röntgenröhren 14 und zwei Röntgendetektoren 15 angeordnet sind. Zur Untersuchung wird ein Patient 16 mit Hilfe einer entlang einer Anlagenachse 17 verschiebbaren Patientenliege 24 in ein Messfeld eingefahren, so dass eine Absorption der Röntgenstrahlung aus unterschiedlichen Projektionswinkeln gemessen werden kann. Zur Steuerung der Anlage dient ein Computer 18, der als Steuer- und Recheneinheit ausgelegt ist. Auf dem Computer 18 laufen Computerprogramme, die eine Steuerung der Computertomografieanlage und eine Auswertung gemessener Daten sowie eine Rekonstruktion der gewünschten tomographischen Bilddaten durchführen.

Insbesondere bei der Übertragung der Detektordaten von den beiden Detektoren 15 auf dem rotierbaren Gantryteil 12 ist es notwendig, eine große Menge von anfallenden Daten kontaktlos zu übertragen. Hierzu wird auf dem rotierbaren Gantryteil 12 und dem feststehenden Gantryteil 13 eine erfindungsgemäße Vorrichtung gemäß der **Fig. 1** bis **Fig. 4** zur kontaktlosen Übertragung elektrischer Signale angebracht, so dass diese zwischen den beiden relativ zueinander rotierbaren Gantryteilen 12 und 13 übertragen werden können.

Auch zur Steuerung von Patiententischen bei bildgebenden medizintechnischen Anlagen kann eine Datenübertragung mit einer Vorrichtung nach **Fig. 5** oder **Fig. 6** erfolgen.

### Bezugszeichenliste

- 1: koaxiales Leiterelement
- 2: koaxiales Koppelleiterelement
- 3: Hochfrequenzsendeeinheit
- 4: Hochfrequenzempfangseinheit
- 5: Dielektrikum
- 6: erstes Trägerelement
- 7: zweites Trägerelement
- 8: erster Außenleiter
- 9: erster Innenleiter
- 10: zweiter Außenleiter
- 11: zweiter Innenleiter
- 12: rotierbares Gantryteil
- 13: feststehendes Gantryteil
- 14: Röntgenröhre
- 15: Röntgendetektor
- 16: Patient
- 17: Analgenachse
- 18: Computer
- 19: Terminierung
- 20: Luftspalt
- 21: Feldlinien
- 22: Drehrichtung
- 23: Bewegungsrichtung
- 24: Patientenliege

## Patentansprüche

1. Computertomografieanlage mit einer berührungslosen Datensignalübertragung, aufweisend:
- mindestens einen längsgeschlitzten Koaxialleiter (1),
- mindestens eine Hochfrequenzsendeeinheit (3), die ein mit einem zu übertragenden Datensignal moduliertes Hochfrequenzträgersignal in den Koaxialleiter (1) einspeist,
- mindestens einen längsgeschlitzten koaxialen Koppelleiter (2), der ausgebildet ist, aus dem Nahfeld des Koaxialleiters (1) das abgestrahlte modulierte Hochfrequenzträgersignal (21) zu empfangen,
- mindestens eine Hochfrequenzempfangseinheit (4), die mit dem Koppelleiter (2) elektrisch verbunden ist und ausgebildet ist, das Datensignal aus dem empfangenen modulierten Hochfrequenzträgersignal zu extrahieren,
- wobei der Koaxialleiter (1) auf einem rotierenden Gantryteil (12) oder einem feststehenden Gantryteil (13) und der Koppelleiter (2) korrespondierend auf dem feststehenden Gantryteil (13) oder dem rotierenden Gantryteil (12) angeordnet ist.

2. Patiententisch für bildgebende medizintechnische Anlagen mit einer berührungslosen Datensignalübertragung, aufweisend:
- mindestens einen längsgeschlitzten Koaxialleiter(1),
- mindestens eine Hochfrequenzsendeeinheit (3), die ein mit einem zu übertragenden Datensignal moduliertes Hochfrequenzträgersignal in den Koaxialleiter (1) einspeist,
- mindestens einen längsgeschlitzten koaxialen Koppelleiter (2), der ausgebildet ist, aus dem Nahfeld des koaxialen Leiterelements (1) das abgestrahlte modulierte Hochfrequenzträgersignal (21) zu empfangen,
- mindestens eine Hochfrequenzempfangseinheit (4), die mit dem Koppelleiter (2) elektrisch verbunden ist und ausgebildet ist, das Datensignal aus dem empfangenen modulierten Hochfrequenzträgersignal zu extrahieren,
- wobei der Koaxialleiter (1) und der Koppelleiter (2) translatorisch relativ zueinander bewegbar angeordnet sind.

3. Vorrichtung nach Anspruch 1 oder 2,
**dadurch gekennzeichnet,**
**dass** der Koaxialleiter (1) und der Koppelleiter (2) ausgebildet sind, ein Hochfrequenzträgersignal größer 10 GHz zu übertragen.

4. Vorrichtung nach einem der vorhergehenden Ansprüche, **gekennzeichnet durch**
- ein erstes Trägerelement (6), in dem der Koaxialleiter (1) ausgebildet ist.

5. Vorrichtung nach Anspruch 4,
**dadurch gekennzeichnet,**
**dass** das erste Trägerelement (6) aus Metall ist und einen ersten Außenleiter (8) des Koaxialleiters (1) bildet.

6. Vorrichtung nach Anspruch 5,
**dadurch gekennzeichnet,**
**dass** das erste Trägerelement (6) im Querschnitt rechteckig ist und ein erster Innenleiter (9) mit einem Dielektrikum (5) in einer Nut des ersten Trägerelements angeordnet ist.

7. Vorrichtung nach einem der vorhergehenden Ansprüche, **gekennzeichnet durch**
- ein zweites Trägerelement (7), in dem der koaxiale Koppelleiter (2) ausgebildet ist.

8. Vorrichtung nach Anspruch 7,
**dadurch gekennzeichnet,**
**dass** das zweite Trägerelement (7) aus Metall ist und einen zweiten Außenleiter (10) des koaxialen Koppelleiters (2) bildet.

9. Vorrichtung nach Anspruch 8,
**dadurch gekennzeichnet,**
**dass** das zweite Trägerelement (7) im Querschnitt rechteckig ist und ein zweiter Innenleiter (11) mit einem Dielektrikum (5) in einer Nut des zweiten Trägerelements (7) angeordnet ist.

## Claims

1. Computed tomography system with a contactless data signal transfer, comprising:
- at least one longitudinally slit coaxial conductor (1),
- at least one radiofrequency transmission unit (3) which feeds a radiofrequency carrier signal, modulated with a data signal to be transferred, into the coaxial conductor (1),
- at least one longitudinally slit coaxial coupling conductor (2) which is embodied to receive the emitted modulated radiofrequency carrier signal (21) from the near field of the coaxial conductor (1),
- at least one radiofrequency reception unit (4) which is electrically connected to the coupling conductor (2) and embodied to extract the data signal from the received modulated radiofrequency carrier signal,
- wherein the coaxial conductor (1) is arranged on a rotating gantry part (12) or a stationary gantry part (13) and the coupling conductor (2) is correspondingly arranged on the stationary gantry part (13) or the rotating gantry part (12).

2. Patient table for imaging medical engineering systems with a contactless data signal transfer, comprising:
- at least one longitudinally slit coaxial conductor (1),
- at least one radiofrequency transmission unit (3) which feeds a radiofrequency carrier signal, modulated with a data signal to be transferred, into the coaxial conductor (1),
- at least one longitudinally slit coaxial coupling conductor (2) which is embodied to receive the emitted modulated radiofrequency carrier signal (21) from the near field of the coaxial conductor element (1),
- at least one radiofrequency reception unit (4) which is electrically connected to the coupling conductor (2) and embodied to extract the data signal from the received modulated radiofrequency carrier signal,
- wherein the coaxial conductor (1) and the coupling conductor (2) are movably arranged relative to one another in a translational manner.

3. Device according to Claim 1 or 2,
**characterized**
**in that** the coaxial conductor (1) and the coupling conductor (2) are embodied to transmit a radiofrequency carrier signal greater than 10 GHz.

4. Device according to any one of the preceding claims, **characterized by**
- a first carrier element (6), in which the coaxial conductor (1) is formed.

5. Device according to Claim 4,
**characterized**
**in that** the first carrier element (6) is made of metal and forms a first outer conductor (8) of the coaxial conductor (1).

6. Device according to Claim 5,
**characterized**
**in that** the first carrier element (6) has a rectangular cross section and a first inner conductor (9) with a dielectric (5) is arranged in a groove of the first carrier element.

7. Device according to any one of the preceding claims,
**characterized by**
- a second carrier element (7), in which the coaxial coupling conductor (2) is formed.

8. Device according to Claim 7,
**characterized**
**in that** the second carrier element (7) is made of metal and forms a second outer conductor (10) of the coaxial coupling conductor (2).

9. Device according to Claim 8,
**characterized**
**in that** the second carrier element (7) has a rectangular cross section and a second inner conductor (11) with a dielectric (5) is arranged in a groove of the second carrier element (7).

## Revendications

1. Système de tomodensitométrie assisté par ordinateur, comprenant une transmission sans contact de signal de données, comportant :
- au moins un conducteur (1) coaxial fendu longitudinalement,
- au moins une unité (3) d'émission de haute fréquence, qui injecte, dans le conducteur (1) coaxial, un signal de porteuse de haute fréquence, modulé par un signal de données à transmettre,
- au moins un conducteur (2) coaxial fendu longitudinalement de couplage, qui est constitué pour recevoir, du champ proche du conducteur (1) coaxial, le signal (21) de porteuse de haute fréquence modulé émis,
- au moins une unité (4) de réception de haute fréquence, qui est reliée électriquement au conducteur (2) de couplage et qui est constituée pour extraire le signal de données du signal de porteuse de haute fréquence modulé reçu,
- dans lequel le conducteur (1) coaxial est monté sur une partie (12) tournante de portique ou sur une partie (13) fixe de portique et le conducteur (2) de couplage est monté de manière correspondante sur la partie (13) fixe de portique ou sur la partie (12) tournante de portique.

2. Couchette de patient pour des installations d'imagerie de la technique médicale, comprenant une transmission sans contact de signal de données, comportant
- au moins un conducteur (1) coaxial fendu longitudinalement,
- au moins une unité (3) d'émission de haute fréquence, qui injecte, dans le conducteur (1) coaxial, un signal de porteuse de haute fréquence, modulé par un signal de données à transmettre,
- au moins un conducteur (2) coaxial fendu longitudinalement de couplage, qui est constitué pour recevoir, du champ proche du conducteur (1) coaxial, le signal (21) de porteuse de haute fréquence modulé émis,
- au moins une unité (4) de réception de haute fréquence, qui est reliée électriquement au conducteur (2) de couplage et qui est constituée pour extraire le signal de données du signal de porteuse de haute fréquence modulé reçu,
- dans laquelle le conducteur (1) coaxial et le conducteur (2) de couplage sont montés mobiles en translation l'un par rapport à l'autre.

3. Dispositif suivant la revendication 1 ou 2,
**caractérisé**
**en ce que** le conducteur (1) coaxial et le conducteur (2) de couplage sont constitués de manière à transmettre un signal de porteuse de haute fréquence plus grande que 10 GHz.

4. Dispositif suivant l'une des revendications précédentes,
**caractérisé par**
- un premier élément (6) de support, qui est constitué dans le conducteur (1) coaxial.

5. Dispositif suivant la revendication 4,
**caractérisé**
**en ce que** le premier élément (6) de support est en métal et forme un conducteur (8) extérieur du conducteur (1) coaxial.

6. Dispositif suivant la revendication 5,
**caractérisé**
**en ce que** le premier élément (6) de support est de section transversale rectangulaire et un premier conducteur (9) intérieur, ayant un diélectrique (5), est disposé dans une rainure du premier élément de support.

7. Dispositif suivant l'une des revendications précédentes,
**caractérisé par**
- un deuxième élément (7) de support, constitué dans le conducteur (2) coaxial de couplage.

8. Dispositif suivant la revendication 7,
**caractérisé**
**en ce que** le deuxième élément (7) de support est en métal et forme un deuxième conducteur (10) extérieur du conducteur (2) coaxial de couplage.

9. Dispositif suivant la revendication 8,
**caractérisé**
**en ce que** le deuxième élément (7) de support est de section transversale rectangulaire et un deuxième conducteur (11) intérieur, ayant un diélectrique (5), est disposé dans une rainure du deuxième élément (7) de support.
